# EUROPÄISCHE PATENTSCHRIFT

(11) **EP 1 203 531 B1**
(45) Veröffentlichungstag und Bekanntmachung des Hinweises auf die Patenterteilung: **26.11.2003**
(21) Anmeldenummer: 01123913.4
(22) Anmeldetag: 06.10.2001
(51) Int. Cl.: A01N 47/44, A01N 25/34, A01N 25/12

(54) **Wasserlösliche chlorhexidinhaltige Zusammensetzungen und deren Verwendung**
Water soluble compositions containing chlorhexidine and their use
Compositions solubles dans l'eau contenant de la chlorhexidine et leur utilisation

(30) Priorität: 21.10.2000 DE 10052322
(43) Veröffentlichungstag der Anmeldung: 08.05.2002
(73) Patentinhaber: Degussa Aktiengesellschaft, 40474 Düsseldorf (DE)
(72) Erfinder: Werle, Peter, Dr., 63571 Gelnhausen (DE); Merz, Friedhelm, 55283 Nierstein (DE); Habrich, Jolanta, 63512 Hainburg (DE)

(56) Entgegenhaltungen:
- EP-A- 1 018 337
- DE-A- 3 443 232
- US-A- 3 888 947

## Beschreibung

Die Erfindung betrifft wasserlösliche chlorhexidinhaltige Zusammensetzungen und deren Verwendung. Die Zusammensetzung enthält Chlorhexidin in Form der freien Base oder in Form eines Chlorhexidin-disalzes.

Chlorhexidin (CHD), chemisch als 1,1'-Hexamethylen-bis-[5.4.chlorphenyl]-biguanid anzusprechen, ist eine stark basisch wirkende Substanz mit nur sehr geringer Wasserlöslichkeit. Durch Umsetzung der Chlorhexidinbase mit Säuren ist eine Vielzahl von ebenfalls schwer wasserlöslichen Salzen zugänglich; wasserlöslich sind dagegen einige Salze von Chlorhexidin mit Zuckersäuren. Chlorhexidinbase und vor allem das wasserlösliche Chlorhexidindigluconat, ein Salz der D(+)-Gluconsäure mit Chlorhexidin stellen bedeutende antibakterielle Substanzen dar, sowohl für den Einsatz im Human- als auch im Tierbereich. Hervorzuheben sind die niedrige Toxizität und Verträglichkeit mit kationischen und nichtionischen Detergentien. Chlorhexidindigluconat (CHD-gluc) ist als 20 gew.-%ige wässrige Lösung im Handel erhältlich. Flüssige chlorhexidinhaltige Formulierungen werden vielfältig modifiziert und als antibakterieller Zusatz in Kosmetika, zur Hautdesinfektion, Wundbehandlung, in der Veterinärmedizin als Euterdesinfektionsmittel und auch zur Oberflächendesinfektion eingesetzt.

Die CHD-Gluconatlösungen unterliegen in ihrer Zusammensetzung und Erscheinung den Anforderungen des Europäischen Arzneibuches bzw. der amerikanischen Pharmakopoe. Eine der Reinheitsforderungen ist der auf 500 ppm begrenzte Gehalt an p-Chloranilin. In Umkehrung der Bildung von CHD (= CHD-Base) aus Hexamethylenbiscyanoguanidin und p-Chloranilin gemäß nachfolgender Gleichung 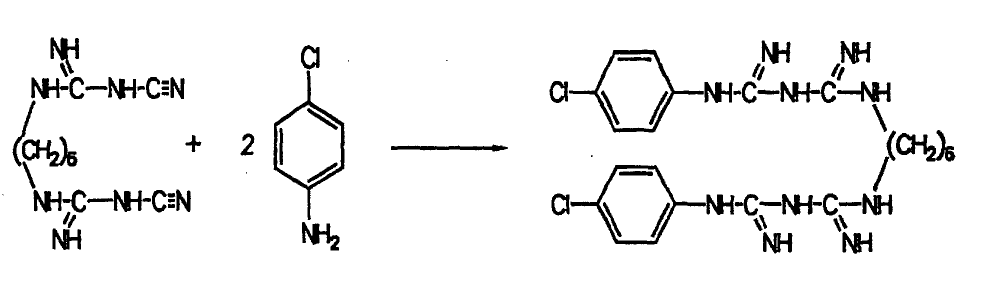 kann bei der Salzbildung der CHD-Base mit D(+) Gluconsäure-δ-lacton, dem inneren Ester der D(+)-Gluconsäure, in wässriger Phase sowie beim Stehenlassen, also Lagern dieser Lösungen, p-Chloranilin reversibel abgespalten werden. Mit zunehmender Lagerzeit kommt es zur Verfärbung der Lösungen in den gelb bis bräunlichen Bereich. Diese Zersetzung von Chlorhexidindigluconatlösungen ist abhängig vom pH-Wert der Lösung und vor allem der Lagertemperatur. Untersuchungen zeigten, dass bei einer Lagerung einer 20 gew.-%igen Lösung Chlorhexidindigluconat bei 40 °C nach etwa einem Monat die zulässigen p-Chloranilinwerte überschritten werden. Auch vielfach verwendete Lösungen, welche CHD-digluconat und andere mikrobiozid wirksame Stoffe enthalten, unterliegen in unterschiedlichem Ausmaß dieser Hydrolyse.

Eine vielfach verwendete Kombination enthält Chlorhexidindigluconat und N-Cetyl-N,N,N-trimethylammoniumbromid (Cetrimide) als Aktivsubstanzen in verdünnten wässrigen oder alkoholischen Lösungen. Auch solche Lösungen weisen eine ungenügende Lagerstabilität auf. Chlorhexidin oder ein Salz davon und Cetrimide oder ähnliche Mikrobiozide enthaltende feste Zusammensetzungen, die in Wasser klar löslich und auch bei höherer Lagertemperatur in sich stabil sind, sind bislang nicht bekannt. Aufgabe der vorliegenden Erfindung ist somit die Bereitstellung derartiger biozider Mischungen, aus denen durch Versetzen mit Wasser gebrauchsfertige Lösungen gewonnen werden können.

Gegenstand der Erfindung ist eine wasserlösliche chlorhexidinhaltige pulver-, granulat- oder tablettenförmige Zusammensetzung, bestehend im wesentlichen aus
(i) einem Salz von Chlorhexidin mit 2 Mol einer Zuckersäure und einem mikrobiozid wirksamen quaternären Ammoniumbromid oder aus
(ii) Chlorhexidin, einer Zuckersäure oder Zuckerlacton, wobei pro Mol Chlorhexidin mindestens 2 Mol Zuckersäure oder Zuckerlacton anwesend sind, und einem mikrobiozid wirksamen quaternären Ammoniumbromid.

Die Unteransprüche richten sich auf bevorzugte Ausführungsformen, insbesondere auf Zusammensetzungen auf der Basis von im wesentlichen Chlorhexidindigluconat und Cetrimide oder Chlorhexidin, Gluconsäure oder Gluconolacton und Cetrimide.

Es wurde gefunden, dass sich ein Gemisch aus festem pulverförmigen Chlorhexidindigluconat und Cetrimide in verschiedenen Verhältnissen, wobei das Gemisch bei Bedarf in eine für die Anwendung geeignete, z. B. granulierte, tabellierte oder pelletisierte Form, überführt worden sein kann, überraschenderweise in Wasser rückstandsfrei wieder auflöst und die Herstellung gebrauchsfertiger oder weiter verdünnbarer Lösungen erlaubt. Dieser Befund war unerwartet, da es in einer Chlorhexidindigluconatlösung in Gegenwart von Bromid-Ionen häufig zur Ausfällung von in Wasser schwer löslichem Chlorhexidindibromid kommt.

Aus dem Löslichkeitsprodukt für Chlorhexidindibromid von L = 9,3x10⁻⁸ mol/l können Grenzkonzentrationen für die in der Lösung vorliegenden Spezies, diprotoniertes Chlorhexidin (= CHD-dikation), Bromid-Ion und Chlorhexidindibromid (= CHD-dibromid) berechnet werden. Eine gesättigte Chlorhexidinbromidlösung sollte danach pro 1 ca. 1400 mg Chlorhexidin-dikation und 450 mg Bromid-Ionen enthalten. Mischungen aus festem Chlorhexidindigluconat und Bromid-Ionen enthaltenden Stoffen, wie Cetrimide, sollten sich daher nur beschränkt in Wasser lösen, bzw. bei IonenKonzentrationen, die die durch das Löslichkeitsprodukt festgelegten Höchstwerte überschreiten, zu Ausfällungen führen. Überraschend aber wurde gefunden, daß feste Mischungen aus Chlorhexidindigluconat und Cetrimide sich in Wasser klar auflösen, ohne dass eine zu erwartende Fällung von CHD-dibromid auftritt, wenn das Löslichkeitsprodukt überschritten wird.

Erfindungsgemäße Zusammensetzungen enthalten als Chlorhexidinsalz ein Salz einer Zuckersäure, wobei das Salz aus dem diprotonierten Chlorhexidin-dikation und zwei Säureanionen der Zuckersäure besteht. Außer dem lange bekannten Chlorhexidindigluconat sind derartige Salze in der EP 1 018 337 A2, deren Offenbarung in diese Anmeldung einbezogen wird, beschrieben. Bevorzugte Salze enthalten das Anion der Gluconsäure, insbesondere der D(+)-Gluconsäure, der Lactobionsäure, D-Galactonsäure, L-Mannonsäure, D-Galacturonsäure, D-Gulonsäure und α-D-Heptagluconsäure. Alternativ hierzu kann die zusammensetzung anstelle eines Chlorhexidinsalzes eine Kombination aus Chlorhexidin (freie Base) und einer Zuckersäure, wie den zuvor genannten, oder Lacton einer solchen, wie D-Galacton-γ-lacton, L-Mannonsäure-γ-lacton, D-Gulonsäure-γ-lacton, D-Gluconolacton, α-D-Heptagluconsäure-γ-lacton, enthalten, wobei pro Mol CHD-Base im allgemeinen mindestens zwei Mol Säure oder Lacton anwesend sind, ein Säure- oder Lactonüberschuss, beispielsweise bis 100 %, insbesondere bis 20 %, aber möglich ist. Derartige Kombinationen bilden beim Auflösen in Wasser CHD-Salze.

Unter den mikrobiozid wirksamen quaternären Ammoniumbromiden sind solche Verbindungen zu verstehen, welche mindestens einen mittel- bis langkettige Alkyl- oder Alkenylrest mit 8 bis 18 C-Atomen aufweisen und bis zu drei gleiche oder verschiedene kurzkettige Alkylreste, die ihrerseits zusätzlich eine Hydroxyl- und/oder Halogengruppe oder/und eine Epoxidgruppe enthalten können. Insbesondere handelt es sich um (C₁₀ bis C₁₆)Alkyl-tri(C₁ bis C₃)alkylammoniumbromid, besonders bevorzugt um N-Cetyl-N,N,N-trimethylammoniumbromid, das unter der Bezeichnung Cetrimide als mikrobiozide Aktivkomponente breite Anwendung in Desinfektionsmitteln findet.

Unter dem Begriff "chlorhexidinhaltig" wird verstanden, dass die Zusammensetzung Chlorhexidin in Form der freien Base oder in Form eines Chlorhexidinsalzes, das seinerseits das diprotonierte Chlorhexidindikation aufweist, enthält.

Der Begriff "im wesentlichen" schließt übliche Hilfsstoffe für mikrobiozide Formulierungen ein. Beispiele hierfür sind oberflächenaktive Stoffe, Tablettensprengmittel, Granulierhilfsmittel und Lösungsvermittler. Derartige Hilfsstoffe können in einer Gesamtmenge von bis zu 20 Gew.-%, insbesondere weniger als 10 Gew.-% und vorzugsweise bis zu 5 Gew.-%, jeweils bezogen auf die Summe der Biozide in der Zusammensetzung, enthalten sein.

Das Gewichtsverhältnis der mikrobioziden Aktivkomponenten ist hierbei beliebig. Üblicherweise enthalten die erfindungsgemäßen Zusammensetzungen Chlorhexidin in Form der freien Base oder des diprotonierten Dikations und ein mikrobiozid wirksames quaternäres Ammoniumbromid, wie Cetrimide, im Gewichtsverhältnis im Bereich von 1:2 bis 1:20, insbesondere im Bereich von 1:5 bis 1:20, jedoch kann auch Chlorhexidin oder ein Salz davon gegenüber dem Ammoniumbromid im Überschuss vorliegen.

Ein vollständig wasserlösliches 1:1 Chlorhexidingluconat-Cetrimide-Gemisch kann nach Auflösung in Wasser bis zu etwa 6 Gew.-% Cetrimide enthalten, da die tolerierbare Cetrimidekonzentration durch die beschränkte Cetrimide-Wasserlöslichkeit (ca. 67 g/l) limitiert wird. Bei einem Mischungsverhältnis von CHD bzw. dem Dikation eines Salzes zu Cetrimide wird mit zunehmendem Cetrimide-Gehalt die in Lösung erreichbare CHD-Konzentration entsprechend erniedrigt. Bei einem Gewichtsverhältnis von 1:20 lassen sich daher klare Lösungen nur mit einem Gehalt von etwa 0,6 % CHD-dikation herstellen, da ansonsten Cetrimide ungelöst verbleibt. Höher konzentrierte Lösungen können aber durch Zusatz von Alkoholen hergestellt werden.

Ein weiterer Gegenstand der Erfindung richtet sich auf ein Verfahren zur Herstellung erfindungsgemäßer Zusammensetzungen. Chlorhexidin und eine Zuckersäure oder Lacton davon werden in einem zur Disalzbildung ausreichenden Molverhältnis, das heißt, 1 zu mindestens 2, mit dem quaternären Ammoniumbromid, wie insbesondere Cetrimide, gemischt. Chlorhexidin und das Ammoniumbromid werden vorzugsweise im Gewichtsverhältnis im Bereich von 1:2 bis 1:20, insbesondere 1:5 bis 1:20, eingesetzt. Zweckmäßigerweise liegen alle Einsatzstoffe bereits pulverförmig vor. Das Mischen kann auch ein Mahlen umfassen. Beim oder nach dem Mischen oder Mahlen kann das pulverförmige Gemisch granuliert, pastilliert oder tablettiert werden, wobei ggf. übliche Granulierhilfsmittel und/oder Tablettensprengmittel zusätzlich eingesetzt werden. Anstelle der Kombination aus Chlorhexidin und einer Zuckersäure oder Lacton davon kann ein daraus gebildetes Chlorhexidinsalz eingesetzt werden. Vorzugsweise werden Chlorhexidindigluconat und Cetrimide im Gewichtsverhältnis von 1:1 bis 1:10 eingesetzt.

Ein technisch gangbarer Weg zur Herstellung von pulverfömigem Chlorhexidindigluconat oder anderen Chlorhexidinsalzen von Zuckersäure besteht in der Abkühlung einer 20 bis 40 gew.%-igen wässrigen Lösung des Salzes, auf Temperaturen von -50 bis -80 °C und Entfernen des Wassers durch Hochvakuumsublimation.

Cetrimide ist als Feststoff käuflich zu erwerben. Die Vermischung beider Komponenten erfolgt durch Homogenisieren in geeigneten Apparaturen.

Bei Verwendung von Chlorhexidinbase (a), einem Zuckersäurelacton (b), wie δ-Gluconolacton, und Cetrimide, ist eine Vormischung oder Salzbildung der Komponenten a und b nicht notwendig. Im richtigen Verhältnis eingesetzt, wird auch hier ein Pulver, das sich in Wasser löst, erhalten. Wegen der notwendigen Reaktionszeit der Base und der Dauer der Hydrolyse des Lactons zur freien Säure, benötigen derartige Systeme aber eine längere Lösezeit.

Die erfindungsgemäßen Zusammensetzungen lassen sich zur Herstellung wässriger oder wässrig-organischer Desinfektionsmittellösungen verwenden. Solche Lösungen können die fertige Anwendungskonzentration an beiden biozid wirksamen Komponenten enthalten oder vor der Anwendung noch weiter verdünnt werden.

Wesentliche Vorteile der erfindungsgemäßen Zusammensetzung sind:
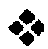 Die hohe Lagerstabilität, da das die Stabilität beeinträchtigende Wasser erst vor der Anwendung zugesetzt wird.
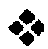 Die leichte Zugänglichkeit und Variationsfähigkeit der Zusammensetzungen.
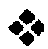 Die leichte Verfügbarkeit von Desinfektionsmittellösungen mit unterschiedlichem Wirkstoffgehalt.

Die nachfolgenden Beispiele sollen die Erfindung näher beschreiben:

### Beispiel 1

### Herstellung von festem Chlorhexidindigluconat

200 g einer 20 %igen (100 g einer 40 %igen) Chlorhexidindigluconatlösung werden in einem 1 1 Kolben vorgelegt und in einem Gemisch aus festem Kohlendioxid in Ethanol ausgefroren. Die ca. -78 °C kalte, kristalline Masse wird anschließend an einer Hochvakuumsublimationsapparatur vom Wasser befreit. Chlorhexidingluconat hinterbleibt als völlig farbloses, pulverförmiges Produkt. Restwassergehalt < 1 %. Beim Erwärmen beginnt es ab ca. 60 °C stark zu sintern, eine klare Schmelze liegt bei 85 bis 90 °C vor.

### Beispiele 2 bis 4

Gemäß den Beispielen 2a bis 4a wurden aus Chlorhexidindigluconat und Cetrimide Chorhexidindigluconat (= CHD-gluc) / Cetrimide Mischungen in den Verhältnissen 1:1, 1:5 und 1:10 hergestellt. Die Feststoffe wurden in Schraubgläser eingewogen und anschließend 30 Minuten homogenisiert.

Anstelle von festem CHD-digluconat wurde in den Beispielen 2b bis 4b eine Mischung aus CHD-Base und δ-Gluconolacton verwendet.

| Beispiel | CHD-gluc [g] | Cetrimide [g] | CHD-Base [g] | δ-Glucolacton [g] |
|---|---|---|---|---|
| 2a | 15 | 15 | - | - |
| 2b | - | 15 | 10.9 | 4.1 |
| 3a | 15 | 75 | - | - |
| 3b | - | 75 | 10.9 | 4.1 |
| 4a | 15 | 150 | - | - |
| 4b | - | 150 | 10.9 | 4.1 |

### Beispiel 5

Lösungen der Mischungen der Zusammensetzungen der Beispiele 2b bis 4b wurden durch Einbringen der gewünschten Menge an Aktivsubstanz in Wasser hergestellt. Die nachfolgende Tabelle gibt die Lösezeiten zur Herstellung von Lösungen mit verschiedenen Konzentrationen wieder.

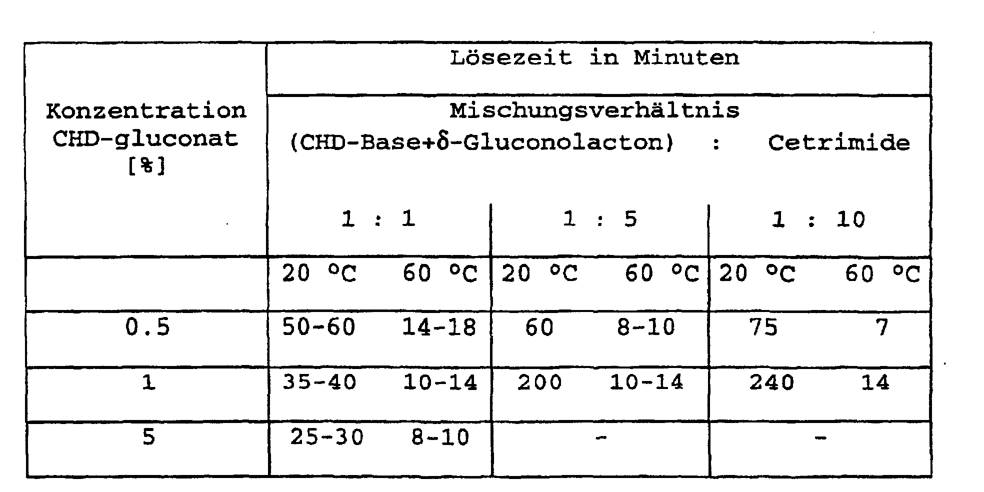

Bei Verwendung von festem, durch Gefriertrocknung gewonnenem, CHD-digluconat verkürzen sich die Lösezeiten wesentlich. Sie liegen bei Raumtemperatur im Bereich von etwa 5 Minuten, wobei die Lösezeit durch die Lösegeschwindigkeit der Cetrimide-Komponente bedingt ist.

Bei Konzentrationen von 1.0 % CHD-digluconat in der Lösung und einem CHD-digluconat/Cetrimide-Verhältnis von 1:1 liegen in der Lösung 10 g CHD-gluconat bzw. 5630 mg CHD-Base sowie 2090 mg Bromid-Ionen vor. Trotzdem tritt keine Ausfällung von CHD-dibromid ein. Ein Zusatz von Alkoholen (n-Propanol; Isopropanol; Ethanol) im Bereich von 1 bis 2 % wirkt stabilisierend. Eine solche Stabilisierung ist bei höheren Wirkstoffkonzentrationen und tiefer Lagertemperatur zweckmäßig. Die erfindungsgemäßen festen, vorzugsweise pulver-, tabletten- oder granulatförmigen Mischungen sind lagerstabil und spalten auch bei höheren Temperaturen kein p-Chloranilin ab.

### Beispiel 6

Die Tabelle gibt einen Vergleich der p-Chloranilin-Abspaltung in der erfindungsgemäßen festen Mischung aus CHD-digluconat und Cetrimide im Gewichtsverhältnis 1:1 bei 40 °C Lagertemperatur und einer 4 %igen wässrigen Lösung hiervon.

| Lagerdauer | Pulver | Lösung |
|---|---|---|
| Start | < 10 | < 10 |
| 1 Monat | < 10 | 23 |
| 3 Monate | < 10 | 38 |
| 6 Monate | < 10 | 55 |

## Patentansprüche

1. Wasserlösliche chlorhexidinhaltige pulver-, granulatoder tablettenförmige Zusammensetzung, bestehend im wesentlichen aus
(i) einem Salz von Chlorhexidin mit einer Zuckersäure, wobei das Salz aus dem diprotonierten Chlorhexidindikation und zwei Säureanionen der Zuckersäure besteht, und einem mikrobiozid wirksamen quaternären Ammoniumbromid oder aus
(ii) Chlorhexidin, einer Zuckersäure oder Zuckerlacton, wobei pro Mol Chlorhexidin mindestens 2 Mol Zuckersäure oder Zuckerlacton anwesend sind, und einem mikrobiozid wirksamen quaternären Ammoniumbromid.

2. Zusammensetzung nach Anspruch 1,
**dadurch gekennzeichnet,**
**dass** sie als mikrobiozid wirksames quaternäres Ammoniumbromid N-Cetyl-N,N,N-trimethylammoniumbromid (Cetrimide) enthält.

3. Zusammensetzung nach Anspruch 1 oder 2,
**dadurch gekennzeichnet,**
**dass** sie Chlorhexidin in Form der Base oder des diprotonierten Dikations eines Chlorhexidinsalzes und Cetrimide im Gewichtsverhältnis im Bereich von 1 zu 2 bis 1 zu 20, insbesondere 1 zu 5 bis 1 zu 20, enthält.

4. Zusammensetzung nach einem der Ansprüche 1 bis 3,
**dadurch gekennzeichnet,**
**dass** sie im wesentlichen aus Chlorhexidindigluconat als Chlorhexidinsalz und Cetrimide als mikrobiozid wirksames quaternäres Ammoniumbromid besteht.

5. Zusammensetzung nach einem der Ansprüche 1 bis 3,
**dadurch gekennzeichnet,**
**dass** sie im wesentlichen aus Chlorhexidin und δ-Gluconolacton, welche im Molverhältnis von 1 zu 2 bis 1 zu 2,2 anwesend sind, und Cetrimide besteht.

6. Verfahren zur Herstellung einer chlorhexidinhaltigen Zusammensetzung gemäß einem der Ansprüche 1 bis 5,
**dadurch gekennzeichnet,**
**dass** man Chlorhexidin und eine Zuckersäure oder Zuckerlacton in zur Disalzbildung ausreichenden Menge oder ein Disalz von Chlorhexidin mit einer Zuckersäure und ein mikrobiozid wirksames quaternäres Ammoniumbromid, jeweils in Pulverform, in einer Menge bis zu 10 Gewichtsteilen, bezogen auf 1 Gewichtsteil Chlorhexidin mischt und/oder miteinander vermahlt und, sofern erwünscht, das Pulvergemisch granuliert oder tablettiert.

7. Verwendung einer chlorhexidinhaltigen Zusammensetzung gemäß einem der Ansprüche 1 bis 5 oder hergestellt nach dem Verfahren gemäß Anspruch 6 zur Herstellung einer wässrigen oder wässrig-organischen, ein Salz von Chlorhexidin mit einer Zuckersäure und ein mikrobiozid wirksames quaternäres Ammoniumbromid enthaltenden Desinfektionsmittellösung.

## Claims

1. Water-soluble, chlorhexidine-containing composition, in the form of a powder, granules or tablets, substantially consisting of
(i) a salt of chlorhexidine with a sugar acid, wherein the salt consists of the diprotonated chlorhexidine dication and two acid anions of the sugar acid, and a microbicidally active quaternary ammonium bromide, or of
(ii) chlorhexidine, a sugar acid or sugar lactone, with at least 2 moles of sugar acid or sugar lactone being present per mole of chlorhexidine, and a microbicidally active quaternary ammonium bromide.

2. Composition according to claim 1,
**characterised in that**
it contains N-cetyl-N,N,N-trimethylammonium bromide (cetrimide) as the microbicidally active quaternary ammonium bromide.

3. Composition according to claim 1 or 2,
**characterised in that**
it contains chlorhexidine in the form of the base or the diprotonated dication of a chlorhexidine salt and cetrimide in a weight ratio in the range of 1:2 to 1:20, particularly 1:5 to 1:20.

4. Composition according to one of claims 1 to 3,
**characterised in that**
it consists substantially of chlorhexidine digluconate as the chlorhexidine salt and cetrimide as the microbicidally active quaternary ammonium bromide.

5. Composition according to one of claims 1 to 3,
**characterised in that**
it consists substantially of chlorhexidine and δ-gluconolactone, which are present in a molar ratio of 1:2 to 1:2.2, and cetrimide.

6. Process for the production of a chlorhexidine-containing composition according to one of claims 1 to 5,
**characterised in that**
chlorhexidine and a sugar acid or sugar lactone, in a quantity adequate for the formation of a disalt, or a disalt of chlorhexidine with a sugar acid and a microbicidally active quaternary ammonium bromide, each in the form of a powder, in a quantity of up to 10 parts by weight, based on 1 part by weight of chlorhexidine, are mixed and/or ground together and, if desired, the powder mixture is granulated or tableted.

7. Use of a chlorhexidine-containing composition according to one of claims 1 to 5 or produced by the process according to claim 6 for the production of an aqueous or aqueous-organic disinfectant solution containing a salt of chlorhexidine with a sugar acid and a microbicidally active quaternary ammonium bromide.

## Revendications

1. Compositions solubles dans l'eau contenant de la chlorhexidine. sous forme de poudre, de granulés ou de comprimés, constituées pour l'essentiel
(i) d'un sel de chlorhexidine avec un acide saccharique, ledit sel étant constitué d'un dication de chlorhexidine diprotoné et de deux anions acides de l'acide saccharique, et d'un bromure d'ammonium quaternaire à activité microbiocide ou
(ii) de chlorhexidine, d'un acide saccharique ou d'une lactone d'acide saccharique, en ayant, pour une mole de chlorhexidine au moins deux moles d'acide saccharique ou de lactone d'acide saccharique. et d'un bromure d'ammonium quaternaire à activité microbiocide.

2. Composition selon la revendication 1.
**caractérisée en ce qu'**
elle contient du bromure de N-cétyl-N,N,N-triméthylamtnonium (cetrimide) à titre de bromure d'ammonium quaternaire à activité microbiocide.

3. Composition selon la revendication 1 ou 2,
**caractérisée en ce qu'**
elle contient de la chlorhexidine sous forme de base ou de dication diprotoné d'un sel de chlorhexidine et de la cetrimide dans un rapport pondéral allant de 1 : 2 à 1 : 20, plus particulièrement de 1 : 5 à 1 : 20.

4. Composition selon l'une des revendications 1 à 3,
**caractérisée en ce qu'**
elle est constituée pour l'essentiel de digluconate de chlorhexidine à titre de sel de chlorhexidine et de cetrimide à titre de bromure d'ammonium quaternaire à activité microbiocide.

5. Composition selon l'une des revendications 1 à 3,
**caractérisée en ce qu'**
elle est constituée pour l'essentiel de chlorhexidine et de δ-gluconolactone présentes dans un rapport molaire allant de 1 : 2 à 1 : 2,2 et de cetrimide.

6. Procédé de préparation d'une composition contenant de la chlorhexidine suivant l'une des revendications 1 à 5.
**caractérisé en ce qu'**
on mélange et/ou on moud intimement de la chlorhexidine et un acide saccharique ou une lactone d'acide saccharique dans une quantité suffisante pour la di-saliflcation ou bien on mélange et/ou on moud intimement un di-sel de chlorhexidine avec un acide saccharique et un bromure d'ammonium quaternaire à activité microbiocide, chacun étant sous forme de poudre, dans une quantité allant jusqu'à 10 parties en poids par rapport à 1 partie en poids de chlorhexidine et, si on le souhaite, on transforme ce mélange pulvérulent en granulés ou comprimés.

7. Utilisation d'une composition contenant de la chlorhexidine suivant l'une des revendications 1 à 5 ou bien préparée selon le procédé suivant la revendication 6 destinée à la préparation d'une solution désinfectante aqueuse ou organique aqueuse contenant un sel de chlorhexidine avec un acide saccharique et un bromure d'ammonium quaternaire à activité microbiocide.
